# EUROPEAN PATENT SPECIFICATION

(11) **EP 4 486 863 B1**
(45) Date of publication and mention of the grant of the patent: **04.11.2026**
(21) Application number: 23708750.7
(22) Date of filing: 28.02.2023
(51) Int. Cl.: C12M 1/00, C12M 1/04, C12M 1/32, C12M 3/00, C12M 3/06

(54) **CULTIVATION DEVICE FOR BIOLOGICAL CELL MATERIAL AND METHOD OF MANUFACTURING THEREOF**
KULTIVIERUNGSVORRICHTUNG FÜR BIOLOGISCHES ZELLMATERIAL UND VERFAHREN ZUR HERSTELLUNG DERSELBEN
DISPOSITIF DE CULTURE POUR MATÉRIAU CELLULAIRE BIOLOGIQUE ET SON PROCÉDÉ DE FABRICATION

(30) Priority: 01.03.2022 EP 22159512
(43) Date of publication of application: 08.01.2025
(73) Proprietor: Medizinische Universität Innsbruck, 6020 Innsbruck (AT)
(72) Inventor: AUSSERLECHNER, Michael, 6020 Innsbruck (AT); HAGENBUCHNER, Judith, 6020 Innsbruck (AT); NOTHDURFTER, Daniel, 6020 Innsbruck (AT)
(74) Representative: v. Bezold & Partner Patentanwälte - PartG mbB
(86) International application number: PCT/EP2023/055011
(87) International publication number: WO 2023/165986

(56) References cited:
- WO-A1-2020/120341
- US-A1- 2018 223 251
- US-A1- 2019 093 077

## Description

### Field of the invention

The present invention relates to a cultivation device being configured for cultivating biological cell material, like e. g. tissue model material. Furthermore, the present invention relates to a method of manufacturing the cultivation device and to methods of using the cultivation device. Applications of the invention are available in the fields of biotechnology, biochemistry and medicine, e. g. for creating and/or investigating cell composites.

### Prior art

In the present specification, reference is made to the following prior art illustrating the technical background of the invention:
[1] A. van den Berg et al. "Personalised organs-on-chips: functional testing for precision medicine" in "Lab Chip" 19, 198, 2019;
[2] G. Trujillo-de Santiago et. al "The Tumor-an-Chip: Recent Advances in the Development of Microfluidic Systems to Recapitulate the Physiology of Solid Tumors in "Materials" 12, 2945, 2019;
[3] J. Hagenbuchner et al. "3D bioprinting: novel approaches for engineering complex human tissue equivalents and drug testing" in "Essays Biochem." 65(3), 417-427, 2021;
[4] H.-W. Kang et al. "A 3D bioprinting system to produce human-scale tissue constructs with structural integrity" in "Nature biotechnology", 34(3), 312-319, 2016;
[5] S. Ji et al. "Complex bioprinting methods" in "APL Bioeng." 5, 011508, 2021;
[6] WO 2020/120341 A1; and
[7] US 2018/223251 A1.

In recent years, *in vitro* tissue models that mimic individual microenvironments of specific human tissue became more and more important for patient tailored therapies, especially in the fields of cancer- or immune therapy (see e. g. [1]). Modelling the tumor-environment holds tremendous potential to better understand the mechanisms how tumors communicate with neighboring, non-transformed cells. Due to individual gene expression patterns and mutations, the mode of interaction between tumor cells and tissue environment differs between tumor types and patients; therefore personalized tumor-tissue models may serve as drug-testing platforms to identify and optimize individual therapy regimens or provide novel insights into the signaling between specific cancer types and their microenvironment.

Another important application of *in vitro* tissue models is the attempt to replace live animals like rodents during drug testing in medical research and development procedures. Besides substantial moral aspects, the species-specific differences in drug transport mechanisms, digestive behavior and especially in liver metabolism can be quite significant from equivalent procedures inside a human body. Moreover, the "living nature" of life animals induces additional uncertainties into a series of measurements; hence, larger sample sizes are needed to separate these influences from the actual measurand of interest.

Currently, there are two predominant ways to utilize appropriate *in vitro* models, comprising two-dimensional (2D) and three-dimensional (3D) cell cultures. Current strategies for the 2D cultivation of living tissue models include one of the following approaches. Firstly, it is known to cultivate one- to two-layer tissue models (e.g., skin equivalents) in so called Boyden chamber inserts on a semi-permeable membrane. These are relatively easy-to-prepare tissue models, which have limitations in terms of lack of substructuring (e.g. no imprinting of vessels), complex manual processes, static culture conditions, difficulties in handling for top/bottom microscopy, and difficulties in standardization.

Secondly, organ on chip systems can be implemented with microfluidic devices where fluidic chips with fine channels are fabricated by photolithography or stereolithography printing. While the channels provide advantages for a medium flow (supply of oxygen and nutrients/growth factors, removal of metabolites), there are limitations resulting from the low layer thickness of tissue (e. g. 50 µm to 150 µm) and a restricted access to the sample embedded in a chip for further analysis. In particular, tissue models obtained with such 2D cultures lack the complexity of physiological, branched and interconnected micro-vessel networks and capillary geometries, which are formed by cells in natural environments.

As a third known approach, organ on membranes represent a combination of the above technologies (see e. g. [2]), wherein a chip with a semipermeable membrane is flowed through by medium and a cell layer is applied to the membrane. As a disadvantage of this concept, tissues are single-layered or consist of only very few cell layers, so that no real 3D structures can be obtained.

On the other hand, 3D cultivation is obtained by 3D bioprinting of cell material that allows the assembly of multilayered, structured and several millimeters thick cell containing hydrogels that resemble natural human tissue much better than 2D cultures (see e. g. [3] to [7]). 3D printing of living human or animal tissue has developed rapidly in recent years and now already enables the printing of living heart and lung models. The tissue equivalents are usually printed into a receptacle cavity within a substrate body, like small containers in fluidic chips, in order to cultivate them further or to perfuse them with nutrient medium like organs before transplantation.

A major challenge in 3D cultivation is to trigger vessel formation and vascularization in the culture volume. To this end, additive-manufactured cell composites including channels have been proposed, which are made by printing a 3D culture in combination with plural filaments of a removable additive substance (e.g. Pluronic, see e. g. [3], [5]). The additive substance is resolved after printing, so that elongated empty spaces are created, which provide vessel-like channels. Despite of the advantages of this vascularization for the supply of nutrients, 3D cultivation still suffers from substantial limitations in terms of stability of cultivation conditions.

Practical tests have shown an unstable connection between cell material including vessel-like channels and external supply tubes, e. g. channels of a chip accommodating the 3D culture. This limitation is also influenced by a drawback of the conventionally used hydrogel components, which typically comprise natural polymers such as collagen and fibrin. These natural polymers are applied due to their high resemblance of physiological extracellular matrix composition, favorable adhesion motives for cell attachment and angiogenesis promoting features natural materials. However, they have a poor mechanical stability and durability during long-term cultivation.

Due to the limited stability, durability and reproducibility of the cultivation conditions, practical applications of current 3D-bioprinted models are restricted to basic research with relatively small cell composite samples and special cell types. For routine applications in medicine and biology, there is a need for a reproducible and easy-to-use platform technology for parallelized long-term culture at physiologic flow conditions in a perfusion system.

As a further general limitation of conventional techniques, also resulting from the low stability of cell cultures, it is not possible to link 3D-bioprinted models of different tissue types in a sequential manner, allowing for instance to study the effect of a medication on a specific organ (e.g., the lung) if the medication has first been altered by a digestive process, such as by the liver, or to study the, possibly toxic, effect of metabolic degradation products of a medication on other organs.

### Objective of the invention

The objective of the invention is to provide an improved cultivation device being configured for cultivating three-dimensional (3D) cell cultures and being capable of avoiding limitations of conventional techniques. In particular, the objective of the invention is to provide the cultivation device with improved stability, durability and/or reproducibility of the cultivation conditions and/or with a capability as a reproducible and easy-to-use platform technology for parallelized long-term culture at physiologic conditions, in particular flow conditions in a perfusion system, and/or with a capability of employing natural polymers as cell matrix material. Furthermore, the cultivation device is to be capable to process multiple cell cultures in a sequential manner. Furthermore, the cultivation device is to be capable to cultivate larger sample. Furthermore, the objective of the invention is to provide an improved method of manufacturing the cultivation device being capable of avoiding limitations of conventional techniques.

### Summary of the invention

The above objective is solved by a cultivation device and a method of manufacturing thereof, comprising the features of the independent claims, respectively. Preferred embodiments and applications of the invention arise from the dependent claims.

According to a first general aspect of the invention, the above objective is solved by a cultivation device, being configured for cultivating biological cell material, comprising a substrate body having an upper surface, a lower surface, at least one receptacle cavity with a bottom wall and a circumferential side wall, and at least one fluid channel in communication with the at least one receptacle cavity, wherein the at least one receptacle cavity has an opening towards the upper surface of the substrate body and is arranged for accommodating the biological cell material.

According to the invention, an inner surface of the side wall is provided with anchoring notches being arranged for holding the biological cell material in the at least one receptacle cavity, and the upper surface of the substrate body has a deepened surface section around the opening of the at least one receptacle, wherein the deepened surface section is adapted for accommodating a cavity lid sheet covering the opening of the at least one receptacle cavity. Optionally, the at least one fluid channel may be provided with a deepened surface section as well.

According to a second general aspect of the invention, the above objective is solved by a method of manufacturing the cultivation device according to the first general aspect of the invention or an embodiment thereof, comprising the step of creating the substrate body with the at least one receptacle cavity, the at least one fluid channel, the anchoring notches and the deepened surface section by subtractive manufacturing from a preformed substrate body workpiece and/or by additive manufacturing.

Advantageously, the invention overcomes the disadvantages of conventional techniques by the combination of 3D bioprinting methods with fluidic chip technology to generate a complex, preferably vascularized tissue-on-a-chip model for mid- and high-throughput applications. The inventive cultivation device provides a platform that combines a standardized production and easy handling of fluidic chips with the complexity of a millimeter-thick, human, vascularized soft tissue equivalent.

The term "biological cell material" refers to a material including at least one of biological cells and cell components thereof. The biological cells may comprise any cell type of human or animal origin. The biological cell material may be composed exclusively of biological cells and/or cell components, or it may comprise biological cells and/or cell components and additionally natural and/or non-natural polymers, like collagen, fibrin, and/or at least one hydrogel. The cells may comprise for instance non-differentiated cells, like stem cells, in particular induced pluripotent stem cells (iPSC) and/or iPSC-derived cells and/or mesenchymal stem cells. Alternatively or additionally, the cells may comprise differentiated cells of any cell type present in human or animal tissues, like e. g. fibroblasts, endothelial cells, mesothelial cells, smooth muscle cells, organ cells, like lung cells or liver cells and/or epithelial cells, from human and/or animal origin. Alternatively or additionally, the cells may comprise spheroid cell bodies. The cells may comprise one single cell type or a mixture of at least two cell types. In particular depending on the application, the biological cell material is indicated also as tissue equivalent, tissue model, cell model, cell composite, cell culture, 3D culture or biological sample in the following.

Preferably, the hydrogel including the cells and/or cell components of the biological cell material comprises at least one natural hydrogel component, like gelatin-methacryloyl (GelMA), alginate, functionalized alginate-YISGR, chitosan, hyaluronic acid, hyaluronic acid metacrylate, collagen, nano-cellulose, silk, synthetic hydrogels, like PEG, PEGDA or PVA, synthetic extracellular proteins, and/or a glycoprotein, like fibrinogen and/or fibronectin. In particular functionalized GeIMA, as a semi-natural polymer, provides advantageous hydrogel properties including tunable mechanical stability, intact adhesion motives, protease degradation sites and fast cross-linking features.

Depending on the considered phase of manufacturing or employing the cultivation device, the hydrogel and/or the biological cell material including the hydrogel is in a liquid (in particular flowable) or in a solidified, e. g. cross-linked condition. The biological cell material is introduced into the receptacle cavity in the flowable condition. Subsequently, the biological cell material is solidified. The solidified biological cell material, in particular the solidified hydrogel thereof, has a permeable structure, i. e. it is capable to be permeated (perfused) by a liquid, like a nutrition solution.

Due to the thickness of the biological cell material in the receptacle cavity (preferably above 200 µm), the biological cell material is a 3D material, i. e. the cells or cell components in the biological cell material experience surrounding conditions and interactions in the volume like in a natural environment, e. g. in a biological tissue. Introducing the biological cell material comprises supplying a liquid filament and/or of the biological cell material into the receptacle cavity. This process is also called printing the biological cell material.

The substrate body (also indicated as fluidic chip or substrate chip) preferably comprises a solid component made of a rigid or slightly deformable (but true in shape) material. The substrate body may be provided by a monolithic (integral) material or a multi-component, e. g. multilayered material, including the at least one receptacle cavity and at least one fluid channel in communication with the at least one receptacle cavity. Preferably, the substrate body has a plate shape with a main extension, which particularly preferred is a substantially planar extension. For reference purposes, it is indicated that the upper and lower surfaces of the substrate body extend in the horizontal direction (x-y-direction), while the normal direction of the upper and lower surfaces is a vertical direction (z-direction). Preferably, the opening of the at least one receptacle cavity faces in the vertical direction, i. e. the at least one receptacle cavity is exposed in z-direction.

Preferably, the substrate body is made of a biologically inert, electrically non-conductive material. Particularly preferred, a plastic material, like e. g. polymethylmethacrylate (PMMA) is used. Alternatively, the substrate body may be made of another solvent-free plastic, e. g. acrylnitrile butadiene styrole (ABS) polymer or polycaprolactone, or another inert material, like PDMS, a ceramic or glass. Advantageously, with the biologically inert, electrically non-conductive material, influences of the substrate body material on the biological material are minimized or exclude.

According to further alternatives, the substrate body material may be made of electrically conductive material or may include electrically conductive components, like electrodes. Advantageously, with the electrically conductive material, the cultivation device may fulfil additional functions. For example, the biological material, e.g. heart muscle cells, can be electrically stimulated, or electrophysiological measurements can be conducted with the biological material in the sample receptacle. The electrically conductive material preferably is a biologically inert metal, like e. g. gold or platinum.

Furthermore, the substrate body material may be made of a material of biological origin, like e. g. polylactic acid (PLA) or cellulose. Advantageously, materials of biological origin have a high compatibility with the biological material in the sample receptacle.

The substrate body may include one single receptacle cavity (single sample embodiment), or an array of at least two, preferably a line or matrix arrangement of receptacle cavities (plural samples embodiment). In case of multiple receptacle cavities, at least two of the receptacle cavities can be fluidically coupled via at least one fluid channel, thus allowing to process multiple cell cultures in a sequential manner. With more than one cavity, the cavities may be fluidically coupled in a serial or parallel configuration, or as a combination of both. Advantageously, this allows a simulation of a sequential implementation of metabolic steps in different cell types. It is further possible to connect multiple separate cultivating devices to a network via interconnecting tubes. For example, a drug can first be digested by liver cells before it hits a tumor followed by a study of the effect of the residual products on kidney cells.

The fluid channel generally comprises a portion, like a line or tube, of the substrate body being capable of guiding a fluid (gas or liquid), in particular supplying the fluid to a receptacle cavity, or withdrawing a fluid therefrom. The fluid channel opens to the receptacle cavity directly and/or via a ring channel, and it couples the receptacle cavity with a fluidic system, e. g. fluid reservoirs for nutrition supply, which may be at least partially included in the substrate body or externally arranged separately from the substrate body.

Preferably, the at least one fluid channel is a radially closed channel extending within the substrate body, particularly preferred along the main extension thereof. Alternatively or additionally, the at least one fluid channel may be provided as a closed or open channel on a surface of the substrate body, optionally extending in a direction deviating from the main extension of the substrate body. Thus, the fluid channels can be located in the substrate body, in the upper surface thereof and/or in the lower surface thereof. Preferably, at least two fluid channels are provided per receptacle cavity, but coupling a single fluid channel with a receptacle cavity is also possible.

The receptacle cavity is a hollow space in the substrate body, which can be indicated as a bowl, compartment, container or cultivation space. A bottom wall of the receptacle cavity is arranged opposite to the opening of the receptacle cavity. The bottom wall preferably extends parallel to the main extension, i. e. in horizontal direction. A circumferential side wall of the receptacle cavity is an inner receptacle wall being provided between the bottom wall and the opening. Preferably, the receptacle cavity has a cylindrical shape, so that the circumferential side wall is a cylindrical surface. The vertical direction of the receptacle cavity is also called axial direction, wherein the circumferential side wall limits the receptacle cavity in radial directions relative to the axial direction. Preferably, the side walls of all of the receptacle cavities are provided with the anchoring notches.

Preferably, the receptacle cavity has an axial depth of at least 5 mm, particularly preferred at least 10 mm to 15 mm, and/or at most 30 mm to 40 mm, particularly preferred at most 50 mm. A cross-sectional dimension of the receptacle cavity, like e. g. the diameter thereof, is at least 5 mm, particularly preferred at least 10 mm to 15 mm, and/or at most 30 mm to 40 mm, particularly preferred at most 50 mm. Advantageously, the receptacle cavity provides sufficient space for cell cultures for lifelike studies on them. It is emphasized that the invention is not restricted to these exemplary ranges. Advantages in particular of the anchoring notches also can be obtained with a larger receptacle cavity being capable of accommodating larger tissue constructs, like for transplantation purposes, e. g. for transplantation of skin or organs. For an adaptation to available laboratory equipment, the cultivation device with the receptacle cavity may be dimensioned like a standard microscope slide (76 mm * 26 mm).

The anchoring notches (or: recess structures, recesses) deepen in radial directions from the inner space of the at least one receptacle cavity into the surrounding substrate body material. Preferably, the anchoring notches have a radially extending retaining profile, which is formed such that material in the sample receptacle is trapped, in particular mechanically anchored, in the anchoring notches. The anchoring notches on the side walls of the receptacles allow hydrogel material including cells to be safely locked inside the receptacle cavity, which allows the material to be cultured in the receptacle cavities for long periods of time without a detachment from the walls, thus ensuring a permanent supply of nutrients and oxygen, as well as the drainage of cellular degradation products. Furthermore, the anchoring notches allow the stable cultivation of larger samples of cell material. Furthermore, cell material including natural polymers with low inherent mechanical stability can be stabilized in the receptacle cavity during cultivation.

The deepened surface section generally is a stepped section, where the surface of the substrate body is deepened compared with the remaining surface. The deepened surface section extends along the edge of the opening of the at least one receptacle cavity and optionally along at least one of the sides, preferably along both sides of the at least one fluid channel. The width of the deepened surface section is preferably selected in a range between 1 mm to 5 mm. Depending on the design of the cultivation device, a smaller or larger width can be provided.

The inventors have found that limitations of the culture stability and changes of the cultivation conditions of conventional 3D cultures are caused by changes of the biological samples in the course of cultivation. 3D cell material deforms during cultivation by the influence of internal cell forces. In the course of tissue cultivation and maturation, the tissue contracts over several days or weeks so that it detaches from fluid channels and can no longer be perfused. Deformation is created as the biological cells (especially fibroblasts, myofibroblasts and muscle cells, but also other cell types) produce extracellular matrix and/or exert mechanical forces, so that the cell material is displaced relative to or even detached from the walls of the receptacle cavity. Therefore, for example during perfusion, a nutrient medium can no longer flow through the printed tissue and the system becomes leaky.

Furthermore, the inventors have found that the displacement of the biological sample relative to the chamber walls can be reduced or even excluded by the anchoring notches in the receptacle cavity and the deepened surface section on the upper surface of the substrate body. Advantageously, both of the anchoring notches and the deepened surface section improve a stable fixation of the biological cell material in the receptacle cavity without deteriorating a perfused cultivation of the biological cell material.

Advantageously, the deepened surface section is capable of fulfilling multiple functions. Firstly, the deepened surface section facilitates an arrangement of a cavity lid sheet which can be provided for closing the at least one receptacle cavity. The lid can be manufactured as a breathable coverage or as a tight foil. Secondly, the cell material may protrude from the receptacle cavity and extend to the deepened surface section. Accordingly, the biological sample can be further stabilized in the receptacle cavity. Furthermore, any material in the deepened surface section, like portions of the cell material and/or the cavity lid, may cover also portions of the fluid channel. The deepened surface section facilitates a sealing of the receptacle cavity and/or the fluid channel(s) connected with the receptacle cavity.

The provision of both of the anchoring notches and the deepened surface section provide the following further particular key qualitative and quantitative advantages of the invention.

The combination of the microfluidic substrate body with 3D bioprinting and the direct connection of the fluid channel(s) in the substrate body to the cell material, in particular bio-printed channels therein allows a reproducible, standardized medium throughput production of fluidic chips for printed multi-cellular tissue equivalents to be integrated. By the anchoring notches, in particular a preferred toothed design, e. g. triangular, rectangular or trapezoidal cross-sectional design thereof, and the resulting chamber structure, the living tissue printed in the receptacle cavity is "interlocked" with the substrate body part during hydrogel polymerization and integrated into the substrate body. Detachment/split bonding to the surface of the tissue equivalent is prevented by the deepened surface section provided adjacent to the receptacle cavity and creating a maximum adhesion area.

With the invention, a stabilized fixation of connections is obtained, thus facilitating efficient perfusion and more stable anchoring of the tissue in the receptacle cavity over long cultivation periods.

The cultivation device allows an efficient, standardized production in medium/high throughput processes and an efficient handling for measuring purposes (e.g. microscopy etc.), as the substrate body does not deform or detach from a preferably provided glass substrate and can also be further cultivated in corresponding automated cultivation systems.

The inventive cultivation device allows a deposition of the cell material in the at least one receptacle cavity using a liquid cell material composite (bioink), which induces a spontaneous formation of vascular vessels. To this end, for instance iPS cells can be differentiated to a mesenchymal stem cell population, which promotes the formation of vascular vessel structures by endothelial cells.

According to a preferred embodiment of the invention, the anchoring notches have an angular shape providing a narrowing of the anchoring notches towards the inner surface of the side wall. According to another preferred embodiment of the invention, the anchoring notches have a step shape providing undercut slit openings of the anchoring notches towards the inner surface of the side wall. Accordingly, the anchoring notches of the side wall preferably comprise undercut recesses in the inner surface of the at least one receptacle cavity. Advantageously, as material is added to the receptacle cavity and the adjacent notches, the material is held in place by the notches as if by a barb. When the cell material in the receptacle cavity changes over time due to deformation and/or biological self-organization processes, the angled and/or stepped anchoring notches fix the cell material, so that it cannot detach from the side walls. The angled shape with the narrowing towards the inner surface of the receptacle cavity prevents that material within the anchoring notches is pulled towards the center of receptacle cavity, so that the cell material does not detach from the wall.

Advantageously, various configurations of the anchoring notches are possible. Preferably, the anchoring notches are distributed in azimuthal direction, i. e. they are successively located along the circumferential direction of the inner side wall of the receptacle cavity. Alternatively, the anchoring notches may be distributed in axial direction, e. g. as a series of ring shaped anchoring notches being successively located along the axial direction of the receptacle cavity. According to further preferred embodiments of the invention, the anchoring notches may be evenly or non-evenly distributed along the side wall. With the even distribution the anchoring notches are evenly spaced along the side wall, while irregular intervals are provided in case of the non-even distribution.

The non-even distribution has particular advantages if the strength of a tensile force within the cell material towards the center of the cavity through the shrinking of the material is not axially symmetric around the center of the cavity, as it may be the case e. g. with muscle cells. This ensures that even if the strength of the force is non-uniformly distributed, the cultured cell material is held in place and fluid channels, like supply tubes, are not violated by shear stresses. Furthermore, all of the anchoring notches may have the same shape and size, or they can be of different sizes and/or different shapes.

Preferably, the anchoring notches extend in a direction perpendicular to the upper and lower surfaces of the substrate body, i. e. parallel to the axial direction of the receptacle cavity. This configuration has advantages for manufacturing the cultivation device. Alternatively, the anchoring notches may have angles other than 90° with respect to the upper and lower surfaces, so that additional fixation forces can be created in an advantageous manner.

According to a further preferred embodiment of the invention, the deepened surface section has a rough finish. Advantageously, the rough finish improves the adherence of cell material and/or a cavity lid sheet on the deepened surface section. In particular, if the deepened surface section is used to span the cavity lid sheet over the receptacle cavity and optionally over the fluid channel(s), the cavity lid sheet material can better adhere to the rough surface, thereby creating a more stable condition for the cultivation device.

According to another advantageous embodiment of the invention, the deepened surface section may have a profile with a deepened trench extending around the opening of the at least one receptacle cavity and opening towards the anchoring notches. Advantageously, the trench additionally retains the cell material in the region covered by the lid.

Preferably, the circumferential side wall may have a circumferential bottom recess being located adjacent to the bottom wall and extending in a radial direction in the substrate body. Advantageously, the circumferential bottom recess provides an additional anchoring effect on the cell material. Particularly preferred, the circumferential bottom recess may have a profile, e.g., a stepped shape, which is formed such that cell material in the sample receptacle is trapped in the circumferential bottom recess.

If, according to a particularly preferred embodiment of the invention, a cross-linked hydrogel is arranged in the at least one receptacle cavity, wherein portions of the hydrogel are included in the anchoring notches, advantages for the cultivation of the biological cell material can be obtained. The cross-linked hydrogel may be arranged as a coating on inner surfaces of the receptacle cavity. Preferably, biological cells or cell components or cell materials or tissue components, like extracellular matrix proteins, e. g. collagen, laminin and fibronectin, in particular parts of the biological cell material to be cultivated, may be included in the hydrogel. Optionally, the inner surface at least one fluid channel can be loaded with a coating of the hydrogel as well, while keeping the flow capability of the at least one fluid channel. As an example, the inner walls of the at least one fluid channel may be coated with extracellular matrix proteins that promote the adhesion of endothelial cells.

Accordingly, the at least one receptacle cavity, including the anchoring notches, can be preloaded with the hydrogel, optionally including the cells or cell components. Preloading may comprise coating the inner surfaces of the receptacle cavity including the anchoring notches or at least partially filling the receptacle cavity including the anchoring notches. In case of coating, the growing of the cell material in the receptacle cavity and anchoring notches during the application of the cultivation device is supported. In case of partially or completely pre-filling, the cultivation device is provided in a prefilled condition, e. g. as a kit for investigating biological cell composite.

Preferred embodiments of the method of manufacturing the cultivation device preferably comprise the steps of depositing a liquid hydrogel in the at least one receptacle cavity, so that the anchoring notches are filled with the liquid hydrogel, optionally including the biological cell material, and cross-linking the hydrogel by at least one of irradiation, temperature setting (heating or cooling) and chemical cross-linking. Cross-linking the hydrogel preferably comprises an irradiation with light having a wavelength in a range from 365 nm to 700 nm. Advantageously, in this wavelength range from UVA to red, appropriate photoinitiators are available that can be used for the polymerization of biological hydrogels.

According to a further advantageous embodiment of the invention, the cultivation device includes a membrane layer providing the cavity lid sheet and being coupled with the deepened surface section for covering the opening of the at least one receptacle cavity. In terms of the inventive method, the membrane layer preferably is coupled with the deepened surface section during or after manufacturing the cultivation device. With the membrane layer, a further stabilization of the cell material in the receptacle cavity is obtained in an advantageous manner.

The membrane layer can be fixed to the entire circumference of the deepened surface section, so that the receptacle cavity is closed. This variant can be employed with prefilled receptacle cavities. If hydrogel is present inside the cavity, the lid sheet material covers it. Alternatively, the membrane layer can be fixed to a part of the circumference of the deepened surface section, so that the receptacle cavity is partially closed but still accessible for cell material supply. In the latter case, the membrane layer preferably is completely fixed after the cell material supply, so that the membrane layer adheres to the entire circumference of the deepened surface section during the cultivation of the cell material.

Preferably, one single membrane layer is provided per receptacle cavity. In case of plural receptacle cavities, at least one of the receptacle cavities, preferably all receptacle cavities, can be provided with a membrane layer. Alternatively, one common single membrane layer covers all receptacle cavities in the substrate body. According to preferred variants, the cavity lid sheet provided by the membrane layer comprises a web of filaments, or a tight material, like a film or foil. If the membrane layer comprises a web of filaments, advantages for gas supply to the cell material, e. g. for adjusting a CO₂ containing environment, and for improving cell adherence are obtained.

In terms of the method of manufacturing the cultivation device, the membrane layer preferably is a spun web of filaments. The membrane layer preferably is made by electrospinning (creating a filament by spinning and irregularly depositing the filament on the substrate by the effect of an electric field). According to preferred embodiments of the invention, the membrane layer is coupled with the deepened surface section by an effect of a chemical solvent and/or by a melting process.

The membrane layer may comprise a synthetic or semisynthetic layer, preferably with a thickness in a range from 100 nm up to 50 µm. The membrane may be composed of fibers with a diameter in a range from 50 nm to 300 nm, so that it is called "nanomembrane" below. With preferred examples, the membrane layer is made of polycaprolactone and/or gelatin or another biocompatible, spinnable material.

Preferably, the membrane layer is created with an electrospinning device, which is configured for pulling threads of the membrane material over the substrate body and deposits them on surface of the substrate body, where the at least one receptacle cavity is exposed. This semi-permeable, non-contact spun "nano-patch" bonds the surface of the living tissue to the surface of the substrate body so that it does not detach from the edge even during contraction.

According to another modification of the invention, further biological cell material may be deposited on the membrane layer. Advantageously, this allows e. g. investigating an interaction of the cell material in the receptacle cavity with the cell material on the membrane layer and to better mimic human tissues that contain thin membrane layers, e. g. the basal lamina in human skin or lung tissue.

According to a further preferred embodiment of the invention, the substrate body may be bound via the lower surface to a carrier substrate, in particular comprising glass and/or plastic, like PMMA, or a compound material with a stiffness similar to glass. In terms of the manufacturing method, it is preferred to bond the substrate body to the carrier substrate and to subject the at least one receptacle cavity and the at least one fluid channel, including the anchoring notches and the deepened surface section, to a plasma treatment. Bonding may be obtained with an adhesive or with a biocompatible adhesive tape (e.g. ARcare 90106NB by Adhesive Research Inc.). Advantageously, the mechanical stability and operational efficiency of the cultivation device is improved by the carrier substrate. Furthermore, the fluid channel(s) can be formed exclusively or additionally in the carrier substrate. As an example, by means of stereolithography 3D printing, it is possible to generate the fluid channel(s) inside the carrier substrate, e. g. made of plastic. The plasma treatment additionally improves the adhesion of biological cell material, e. g. tissue and/or muscle cells, and thus also anchors the cells responsible for the shrinkage of the biological sample to the inner walls and anchoring notches.

Subtractive manufacturing comprises providing the preformed substrate body workpiece and creating the at least one receptacle cavity with associated anchoring notches and deepened surface section, as well the at least one fluid channel by material removal. The material removal preferably comprises laser beam-based cutting, in particular engraving. Laser beam-based engraving has advantages in terms of creating nanostructured surfaces with improved adhesion properties for the biological cell material. The laser intensity, laser pulse frequency and the speed of movement of the laser relative to the substrate body can be selected in dependency of the desired structures to be created. Increasing the laser pulse frequency results in smoothening the surface. A low laser pulse frequency, in particular being executed with a pulsed laser having a repetition frequency below 400 Hz, preferably a repetition frequency between 250 Hz and 400 Hz, is preferred for producing a rough surface finish.

Alternatively or additionally, the material to be subtracted from the preformed substrate body workpiece can be removed by mechanical and/or chemical processing methods, such as milling, etching or filing. A combination of mechanical and/or chemical processing methods with additional laser treatment, in particular to obtain the desired surface structure, is also possible.

Additive manufacturing comprises e. g. injection molding, mold casting, deep drawing, stereolithographic methods or holographic volume 3D printing (where the structure is not generated layer by layer, but in the polymer by focusing photons e.g. by 2 photon polymerization) or layer-by-layer 3D printing. Subsequent processing of the surface by a pulsed laser can additionally modify the surface texture. In particular, a rough finish can be produced within the deepened surface section(s).

According to another particularly preferred embodiment of the invention, at least one hollow vessel structure is prepared in the hydrogel by depositing a vessel-shaped sacrificial material in the hydrogel, wherein the sacrificial material is coupled with the at least one fluid channel, and removing the sacrificial material after cross-linking the hydrogel, so that the at least one hollow vessel structure is formed. The sacrificial material may comprise e. g. the polymer Pluronic F127 (trade name). Alternatively, other fugitive substances may be used that serve as support material and can be removed, e.g., by temperature change (gelatin, low melting agarose).

Alternatively, the at least one hollow vessel structure may be printed by an additive printing technique, like e. g. stereolithography or holographic printing methods, in the biological material. Advantageously, the at least one hollow vessel structure can be formed without sacrificial support material in this case.

Preferably, inner surfaces of the at least one fluid channel and the at least one hollow vessel structure are covered with at least one of biological cells, collagen, laminin and fibronectin. The sacrificial material is deposited in liquid state by a deposition technique allowing a localized arrangement of the sacrificial material on or in the hydrogel. The deposition technique may comprise e. g. direct dispensing, extrusion printing, droplet dispensing or spraying.

Preferred applications of the invention comprise at least one of cultivating biological cells, organ-on-chip processes, self-organization of composites of biological cells, ripening of biological cells, investigating degeneration processes of biological cells, testing pharmaceutically active substances (e. g. drug testing), creating multi-tissue/organ models, investigating wound healing processes, creating biosensors, biohazard detection, parasitology investigations, creating barrier models (e. g. blood-brain barrier, skin, intestine, lung), in vitro vaccination processes by implementation of antigen-presenting cells, antigen and vaccine screening, mechanobiological investigations, investigations of living technical circulation systems, creating tumor environments, investigations of tumor vascularization, metastasis and adhesion of tumor metastases, investigations of aging processes, and investigations of tissue-specific metabolism, and methods of disease modelling.

The above preferred examples show that the applications of the invention are manifold and in particular coincide with the applications for all other organ-on-chip technologies. The advantage of the invention is the possibility of long-term cultivation of perfused cell material, e. g. tissue pieces, so that self-organization, maturation processes, but also slow degeneration processes can be studied. In addition, mechanobiological phenomena can be studied in 3D tissue equivalents, which correspond much better to living tissue than, for example, cell monolayers on semipermeable membranes.

Features disclosed in the context of the cultivation device and embodiments thereof also represent preferred features of the inventive method of manufacturing the cultivation device and embodiments of the method. The aforementioned aspects and inventive and preferred features, in particular with regard to the configuration of the cultivation device as well as the dimensions and compositions of individual components being described in relation to the cultivation device, also apply for the method. The preferred embodiments, variants and features of the invention described above are combinable with one another as desired.

### Brief description of the drawings

Further details and advantages of the invention are described in the following with reference to the attached drawings, which schematically show in
- Figure 1:: a perspective overview of features of preferred embodiments of the cultivation device according to the invention;
- Figure 2:: an illustration of features of preferred embodiments of the method of manufacturing the cultivation device according to the invention;
- Figures 3 and 4:: illustrations of creating a cavity lid sheet;
- Figures 5 and 6:: perspective views of further embodiments of the cultivation device according to the invention;
- Figures 7 and 8:: top views of further embodiments of the cultivation device according to the invention, including multiple receptacle cavities;
- Figure 9:: illustrations of creating a cavity lid sheet; and
- Figures 10 and 11:: perspective views of further embodiments of the cultivation device according to the invention.

### Preferred embodiments of the invention

Features of preferred embodiments of the invention are described in the following with particular reference to the configuration of the cultivation device and the manufacturing thereof, in particular with regard to the provision of the receptacle cavity with the anchoring notches and the deepened surface section. Further features of the cultivation device, in particular with regard to the implementation as a fluidic chip and the coupling with further fluidic components, like fluid reservoirs and/or pumps, valves, sensors, and/or monitoring devices, are not described as they are known as such from prior art. Furthermore, features of cultivating biological cells and adjusting cultivation and/or perfusion conditions, like the supply of nutritions and/or differentiation factors, are not described as they are known as such from prior art as well.

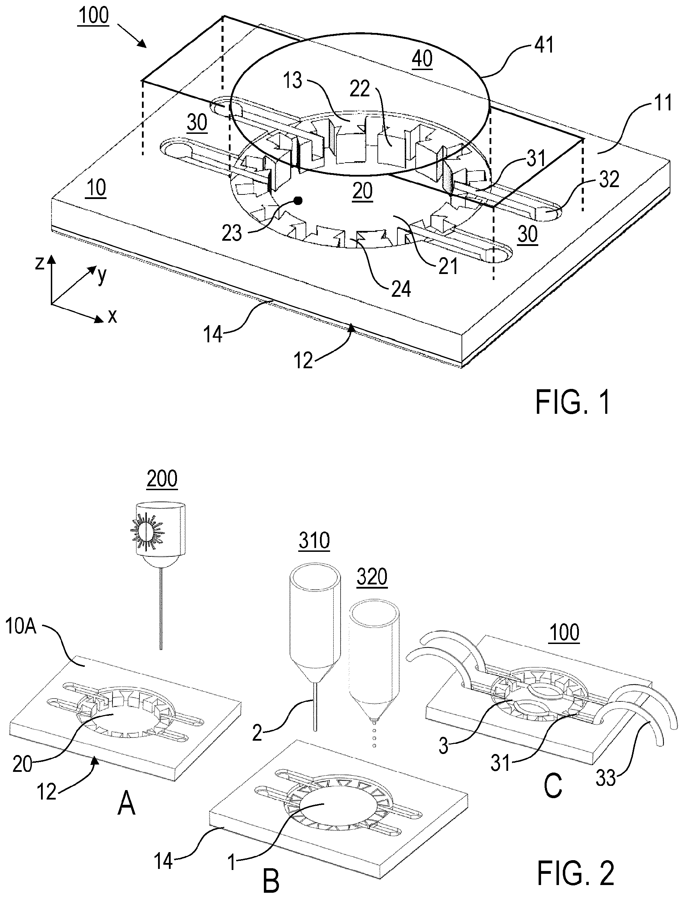

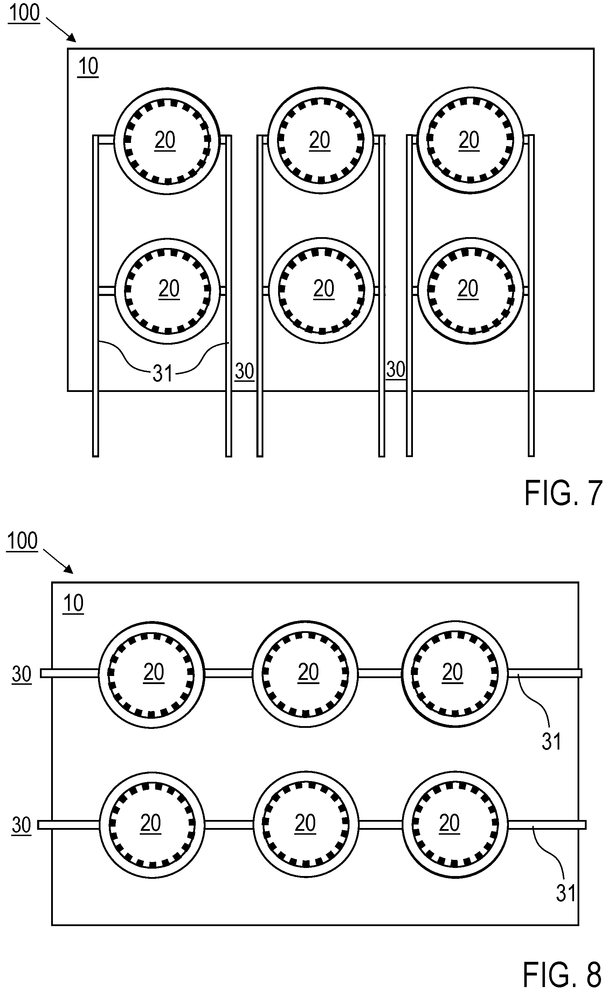

Figure 1 illustrates a first embodiment of the cultivation device 100 according to the invention, including the substrate body 10, the receptacle cavity 20, fluid channels 30 and the cavity lid sheet 40. The receptacle cavity 20 is adapted for accommodating biological cell material (not shown in Figure 1, see Figure 2B). Figure 1 illustrates an embodiment of the cultivation device with a single receptacle cavity 20. The invention is not restricted to this configuration, but rather can be implemented with multiple receptacle cavities, e. g. as shown in Figures 7 and 8. With the illustrated example, the fluid channels 30 comprise four fluid lines 31, each being in fluid communication with the receptacle cavity 20 and having a port section 32 (see Figure 1) for coupling the receptacle cavity 20 via tubes 33 (see Figure 2C) with further components of the fluidic system (not shown). With alternative embodiments, another number of fluid lines, including a single fluid line, may be provided per receptacle cavity 20.

The substrate body 10 is a cuboid-shaped plate with an upper surface 11 and a lower surface 12 extending parallel to the horizontal directions (x-y-plane). The receptacle cavity 20 is created as a through-hole through the substrate body 10. At the upper surface 11, the receptacle cavity 20 has an opening 23, where the inner space of the receptacle cavity 20 is exposed to the surrounding or covered with the cavity lid sheet 40. At the lower surface 12, a carrier substrate 14 is bonded to the substrate body 10. The carrier substrate 14 is made of e. g. glass. With the carrier substrate 14, the receptacle cavity 20 is closed towards the lower surface 12 of the substrate body 10.

The receptacle cavity 20 is formed by a bottom wall 21 and a side wall 22 including the inventive anchoring notches 24. The bottom wall 21 is provided by an exposed portion of the carrier substrate 14. The side wall 22 is provided by the material of the substrate body 10. The vertical direction (z-direction) defines an axial direction of the receptacle cavity 20.

The anchoring notches 24 comprise recesses in the inner side wall 22 of the receptacle cavity 20. Each of the anchoring notches 24 extends in a radial direction in the x-y-plane. Furthermore, each of the anchoring notches 24 extends over the entire thickness of the substrate body 10 in axial direction (z-direction). The anchoring notches 24 are evenly distributed with equal angular spacings along the circumference of the side wall 22. Alternatively, the anchoring notches 24 can be distributed non-evenly and/or with varying shapes and sizes (not shown).

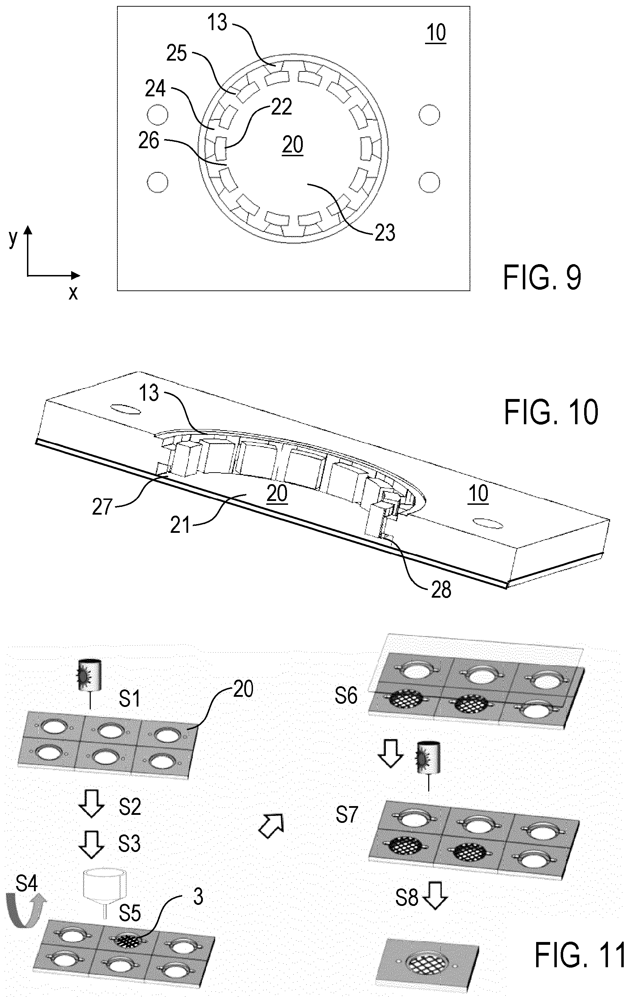

As shown in Figure 1, the anchoring notches 24 preferably have an angular shape creating an inner profile of the anchoring notches 24. The anchoring notches 24 are shaped such that the azimuthal width thereof narrows in radial direction towards the center of the receptacle cavity 20, in particular towards the inner side wall 22. According to the illustrated example, the anchoring notches 24 have a cross-sectional shape similar to a thick arrow, i. e. the inner profile has a narrowing portion and a portion with constant cross-section. The invention is not restricted to this illustrated shape. Alternatively, as further variants, the profile of the anchoring notches 24 can be formed such that the narrowing portion extends over the entire depth of the anchoring notches 24, or the profile of the anchoring notches 24 can be formed like a saw-tooth profile or with a step shape (see Figures 9, 10).

At the upper surface 11, the substrate body 10 has a deepened surface section 13. The deepened surface section 13 surrounds the opening 23 of the receptacle cavity 20 and includes the exposed profiles of the anchoring notches 24. According to the cylindrical shape of the receptacle cavity 20, the deepened surface section 13 has a shape of the ring circle with a width larger than the depth of the anchoring notches 24. With an alternative embodiment, the deepened surface section 13 can have a profile as illustrated in Figures 9 and 10.

Preferably, as shown in Figure 1, the deepened surface section 13 is extended along the fluid channels 30 as well, so that the upper surface 11 is deepened on both sides of the fluid lines 31 and around the port sections 32.

With a practical example, the substrate body 10 has a thickness of 3 mm and an area of 21 mm * 26 mm. The inner diameter of the receptacle cavity 20 is e. g. 10 mm, while about 10 to 20 anchoring notches are provided, each having a radial depth of 1.5 mm and an azimuthal width of about 1.5 mm. The fluid channels 30 have a depth of 2 mm and a length between the receptacle cavity 20 and the port sections 32 of 4 mm. The depth of the deepened surface section 13 is e. g. 400 µm.

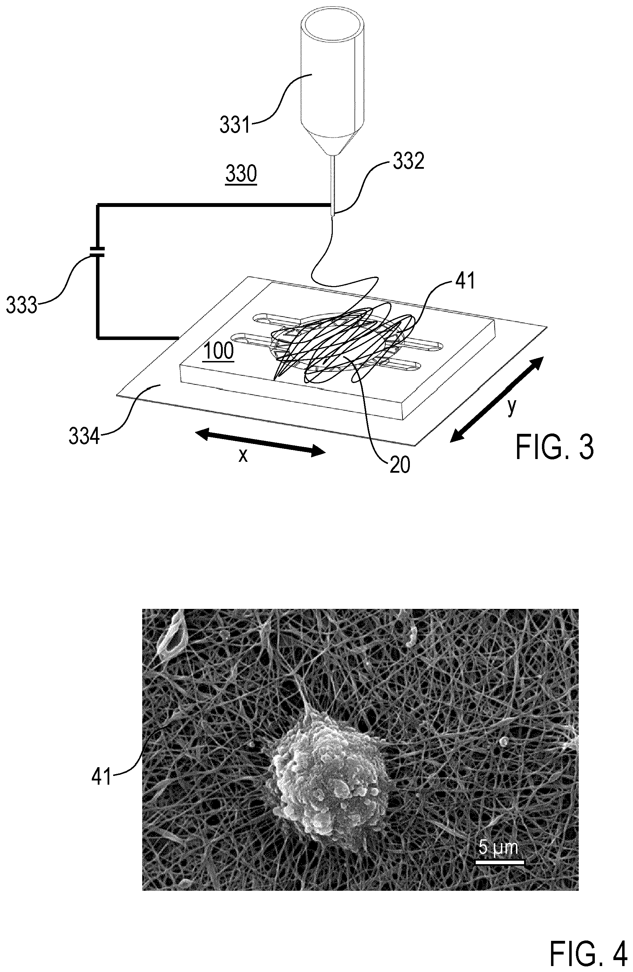

Figure 1 illustrates the cavity lid sheet 40 as a preferred feature of the cultivation device 100 according to the invention. For illustrative purposes, the cavity lid sheet 40 is shown with a distance above the upper surface 11. With the practical application, the cavity lid sheet 40 is arranged directly on the cultivation device 100, in particular on the biological cell material in the receptacle cavity 20. Depending on the application of the cultivation device 100, the cavity lid sheet 40 is provided on a pre-filled cultivation device 100 (with the receptacle cavity 20 containing biological cell material), or it is created after printing the biological cell material into the receptacle cavity 20. Creating the cavity lid sheet 40 is described in an exemplary manner with reference to Figures 3 and 4 below.

Figure 2 schematically illustrates steps of the method of manufacturing the cultivation device according to the invention, e. g. the cultivation device 100 of Figure 1. With the illustrated example, reference is made to manufacturing the cultivation device 100 by a subtractive method.

Firstly, the substrate body 10 is formed from a plate-shaped substrate body workpiece 10A, e. g. an acryl plate with a size of the substrate body 10 to be obtained. The receptacle cavity 20 with the anchoring notches 24 (not shown in detail) and the fluid channels 30 as well as the deepened surface section 13 are created by laser processing, in particular laser cutting and/or laser engraving (see Figure 2A), with a laser apparatus 200. The laser apparatus 200 comprises e. g. the laser system 8000 Laser System Mini (manufacturer: Epilog Laser Systems, Golden, CO, USA). Operation conditions of the laser apparatus 200 are selected in dependency on the material to be processed and the structures to be created. For providing a rough finish of the deepened surface section 13, the laser apparatus 200 is operated with a repetition frequency of about 300 Hz. Figure 2A shows the situation after completion of the receptacle cavity 20 and the fluid channels 30.

Subsequently, a carrier substrate 14 is bonded to the lower surface 12 of the substrate body 10. As an example, the carrier substrate 14 comprises a glass sheet with a size equal to the size of the lower surface 12 of the substrate body 10 and a thickness of e. g. 100 µm. Alternatively, the carrier substrate 14 may comprise a film being adhered to the lower surface 12.

After bonding the substrate body 10 with the carrier substrate 14, the inner surfaces of the substrate body 10 are coated with a hydrogel 2, like GelMA. To this end, a liquid volume of the hydrogel 2 is supplied with a liquid pump device 310, and the liquid hydrogel 2 can be distributed on the inner surfaces of the substrate body 10 by spraying during the printing process directly from the print head also into the anchoring notches and/or by moving, in particular swiveling with an orbital shaker, the substrate body 10. After contacting the inner surfaces of the substrate body 10, the hydrogel 2 dries and/or cross-links, so that a coating with a thickness of e. g. 100 µm is formed. This lowest hydrogel layer preferably contains biological cell material and particularly preferred has the same composition like the remaining material in the sample receptacle. Subsequently, the biological cell material 1 to be cultivated is added to the coated substrate body 10 (Figure 2B).

The biological cell material 1, comprising the same or a modified hydrogel with embedded biological cells, is added by employing another liquid pump device and/or the droplet dispenser device 320. For example the 3D Discovery BioSafety bioprinter (manufacturer: Regenhu, Switzerland) is used for printing the biological cell material. Alternative deposition techniques comprise micro-jet printing, nebulizing by the effect of ultrasound, or printing droplets with needle printers. The biological cell material 1, like e. g. stem cells in alginate, is filled into the inner space of the receptacle cavity 20 and the anchoring notches. The hydrogel included in the biological cell material can then be cross-linked by irradiation, temperature setting, e. g. heating or chemical cross-linking, depending on the composition of the hydrogel. This step polymerizes the hydrogel and locks it together with the biological cells to the anchoring notches. The exact hydrogel composition is selected in dependency on specific requirements like the desired mechanical strength of the cross-linked material, e. g. based on previous tests.

Simultaneously with the introduction of the biological cell material, the vessel-shaped sacrificial material 3 is deposited in the biological cell material, as schematically shown in Figure 2C. The sacrificial material 3 also may be printed into the fluid lines 31 and optionally may be introduced into the tubes 33. The sacrificial material comprises e. g. Pluronic F-127, as described in [3]. The sacrificial material 3 provides a schematically shown network of filaments with cross-sectional dimensions like vessels and vascular structures in the biological cell material 1 to be cultivated. Furthermore, the sacrificial material 3 is coupled with the fluid lines 31 of the fluid channels. After cross-linking the hydrogel, the sacrificial material 3 is removed by dissolution, resulting in a hollow vessel and vascularization structure.

Figures 3 and 4 illustrate the formation of the cavity lid sheet 40 as a mesh-like membrane layer 41 by electro-spinning. The provision of a mesh-like membrane layer 41 has particular advantages in terms of providing an increased surface for efficient cell attachment and cell migration. The spinning apparatus 330 comprises a syringe driver 331 containing a polymer solution, a spinning nozzle 332, a high voltage source 333 and a conductive platform 334. The conductive platform 334 is movable in x- and y-directions, wherein the movement in x-direction preferably is a stepwise movement and slower than in y-direction. Additionally or alternatively, the syringe driver 331 may be movable in x- and y-directions. As a further alternative to the illustrated configuration, instead of the conductive platform 334, two elongated electrodes can be placed along the cultivation device and the high voltage can be switched back and forth between these two electrodes. Since no speed limitations are imposed with this embodiment, advantages in terms of an even better parallel alignment of the filaments can be achieved. The polymer solution includes biomolecules, such as gelatin, collagen etc., dissolved in an organic solvent. Cell adhesion for the cells to be added to the membrane layer 41 can be improved by adding peptids or proteins, like ECM proteins to the polymer solution.

With the syringe driver 331, the polymer solution is supplied to the spinning nozzle 332 with a pressure. A high voltage of e. g. 8.5 kV is applied with the high voltage source 333 between the spinning nozzle 332 and a conductive substrate 334. Due to the electric field created by the high voltage supply, a jet stream of the polymer solution forms at the spinning nozzle 332. The pressure of the polymer solution and the high voltage preferably are adjusted such that a Taylor cone is formed at the nozzle opening, with the jet stream at the tip of the cone.

The concentration of the polymer solution is adjusted such that the material leaving the spinning nozzle 332 cross-links and dries during the flight towards the substrate. Accordingly, nano-fibres are created which form a nano-mesh on the substrate. The nano-mesh may comprise an irregular distribution of the nano-fibres or an at least partially oriented arrangement of the nano-fibres. According to the movement of the conductive substrate 334, a nano-fiber 4 is not only deposited on the conductive platform 334 but is drawn directly over the cultivation device 100 and the hydrogel in the sample receptacle 20. The fast movement of the conductive substrate 334 in y-direction causes a preferential orientation of nanofibers in y-direction. The fibre diameters can be set for instance in a range from 50 nm to 150 nm in case of gelatin fibres. The thickness of the membrane layer 41 depends on the spinning time, and it may amount up to e. g. 30 µm. After completing the spinning, the cultivation device 100 with the covering membrane layer 41 is transferred from the conductive substrate 334 to an application site.

After creating the cavity lid sheet 40 by spinning the membrane layer 41, biological cell material can be applied to the upper exposed surface of the cavity lid sheet 40. As shown with the exemplary electron-microscopy image of Figure 4, single cells, for instance keratinocytes, adhere at the surface of the membrane layer 41 by interactions between the cells and the filaments.

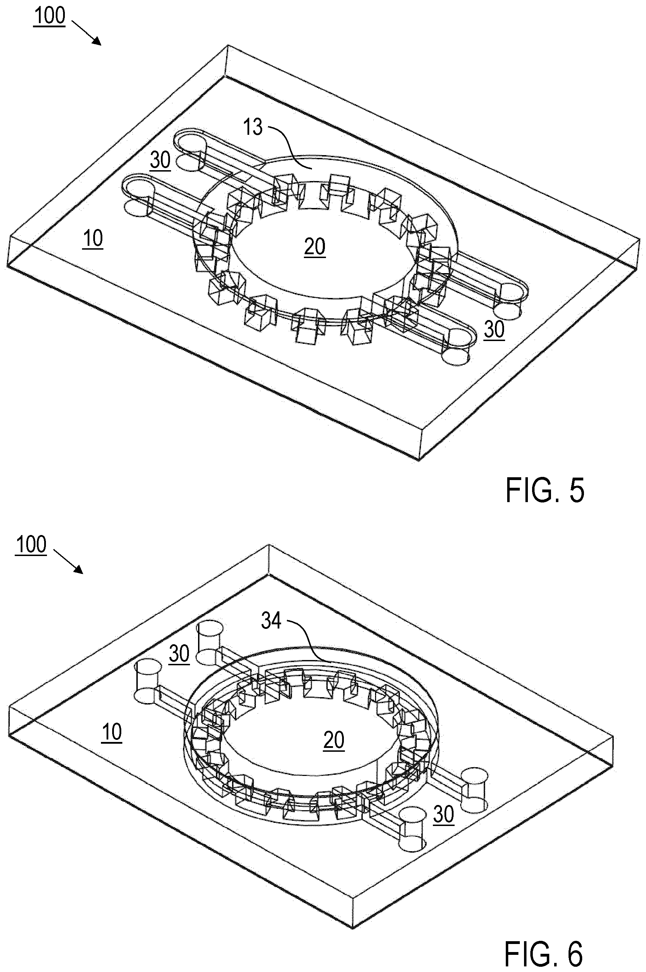

Figures 5 and 6 illustrate further alternative embodiments of the cultivation device 100 (optional cavity lid sheet not shown), which are similarly configured like the embodiment of Figure 1, in particular with the substrate body 10, the receptacle cavity 20 and the fluid channels 30. Deviating from Figure 1, the embodiment of Figure 5 has a broader deepened surface section 13. The radial width of the deepened surface section 13 is about twice the size of the radial depth of the anchoring notches 24 or more. With the broader deepened surface section 13, an improved adherence of the cavity lid sheet and/or the biological cell material is obtained.

With the embodiment of Figure 6, a ring channel 34 is provided additionally to the fluid lines 31. The ring channel 34 is created in the substrate body. The fluid lines 31 and the anchoring notches 24 are fluidically coupled with the ring channel 34. Accordingly, the anchoring notches 24 are open toward the inner space of the receptacle cavity 20 and toward the ring channel 34. With the embodiment of Figure 6, particular advantages in terms of an even perfusion of the biological cell material in the receptacle cavity 20 are obtained.

Figure 7 and 8 illustrate further embodiments of cultivation devices 100, wherein multiple receptacle cavities 20 with anchoring notches are provided in the substrate body 10. Each of the receptacle cavities may have separate fluid lines as shown in Figures 1, 5 and 6. Alternatively, receptacle cavities 20 can be fluidically coupled via shared fluid lines 31 of the fluid channels 30. As shown in Figure 7, groups of receptacle cavities 20 are coupled in parallel via common fluid lines 31 with common perfusion fluid reservoirs (not illustrated). According to Figure 8, groups of receptacle cavities 20 are coupled via fluid lines 31 in a sequential manner so that the perfusion liquid flowing through one of the receptacle cavities is supplied to the subsequent receptacle cavity 20. According to further alternatives, networks of fluid lines including receptacle cavities can be formed.

In Figures 9 and 10, further advantageous features of embodiments of the inventive cultivation device are illustrated. The top view of the substrate body 10 in Figure 9 shows a modified shape of the anchoring notches 24, each radially extending with a retaining profile for trapping material in the receptacle cavity 20. The retaining profile is formed with a step shape 25 providing undercut slit openings 26 of the anchoring notches 24. The slit openings 26 open towards the inner surface of the side wall 22. Cell material is trapped by the undercut slit openings 26, like with the profiles of the anchoring notches 24 shown in Figure 1. With this embodiment, the anchoring notches 24 preferably have an angular shape narrowing in radial direction, as shown in Figure 9, or a straight shape, as shown in Figure 10 (sectional view).

As a further optional feature, the deepened surface section 13 may have a profile in the x-y plane with a deepened trench 15 extending in azimuthal direction around the opening 23 of the receptacle cavity 20, as shown in Figures 9 and 10. The deepened trench 15 directly communicates with the anchoring notches 24 so that ring of material, like e.g. cell material and/or biomatrix material, may be polymerized out in direction connection with the material in the in the anchoring notches 24 and the receptacle cavity 20. This ring of material provides an additional retaining function stabilizing the cell material in the receptacle cavity 20.

Additionally or alternatively, the circumferential side wall 22 may have a circumferential bottom recess 27 being located adjacent to the bottom wall 21 and extending in a radial direction in the substrate body 10. Preferably, the circumferential bottom recess 27 has a step-shaped profile 28, so that a structure similar to the deepened trench 15 is formed. With the circumferential bottom recess 27, cell material in the receptacle cavity 20 is trapped in axial direction (z-direction), while the optional step-shaped profile 28 additionally traps the cell material in radial directions (x-y directions).

By the profiled deepened surface 13 with the trench 15 and the circumferential bottom recess 27 with the step-shaped profile 28, annular retaining structures are formed at the top and bottom so that anchorage extensions are embedded directly in the cell material. The retaining structures may be formed e.g., by laser engraving. In particular, by annular retaining structures at the top and bottom where anchors do not reach the bottom, a degradable substance is allowed to generate a cavity under the cell material. Accordingly, the cell material hangs in the chip (3D anchored) and can be supplied with medium over the entire surface, including from below. In addition, pressure changes (e.g. pulsating flow of medium or different air pressure in skin equivalents) can move the cell material and thus stimulate it mechanically.

Figure 11 shows an example of a workflow for creating a vessel-shaped sacrificial material by additive printing technique. With step S1, the substrate body 20 is prepared by laser engraving and/or cutting receptacles into a PMMA plate from top and bottom sides. After an oxygen plasma treatment (step S2) for charging the plate surface and coating (step S3) a biopolymer, like e.g., collagen, onto the portions of the plate surface, which form the receptacles, the substrate body plate is turned (step S4) and the degradable vessel-shaped sacrificial polymer material 3, e.g., water soluble PVA (polyvinylalcohol) is printed (step S5) into engraved, deepened areas of a bottom side of the substrate body plate. Subsequently, a transparent PMMA foil is fixed on the bottom side (step S6) and the substrate body plate is cut, e.g., by laser cutting, into single slices, each with one receptacle cavity (step S7), followed by an oxygen plasma treatment (step S8) to charge the surface of the PMMA foil, improve wetting, remove solvents and sterilize the completed slide.

In summary, the invention provides a method for the medium-throughput production of 3D-bioprinted tissue equivalents embedded into a preferably laser-manufactured acryl/glass chip, where engraved channels are directly connected to conduits in the tissue equivalent during the printing process. The anchoring notches provide specific macroscopic and microscopic structures in the fluidic chip design that integrate the bioprinted tissue into the substrate body to prevent the detachment of living tissue from the walls of the plastic chip during post-processing. The combination of preferably laser-based surface treatment and electrospun semisynthetic nano-membranes connects and stabilizes the tissue within the plastic parts of the substrate body. With these tissue-on-chip models, the inventors could show that drug testing can be directly performed and studies on physiology, cell biology, mechanobiology, and morphogenesis can be executed sequentially or in parallel.

In practice, the inventive cultivation device has been used for investigating the extracranial solid tumor neuroblastoma, which develops in early childhood and still has a poor prognosis. One strategy to increase cure rates is the identification of patient-specific drug responses in tissue models that mimic the interaction between patient cancer cells and tumor environment. The inventive cultivation device has been used for developing a perfused and micro-vascularized tumor environment model that is directly bioprinted into the receptacle cavity. A gelatin-methacrylate/fibrin-based matrix containing multiple cell types mimics the tumor-microenvironment that promotes spontaneous micro-vessel formation by embedded endothelial cells. Fluid channels are coated with endothelial cells *post* printing to form a vessel - tissue barrier. The tissue model thereby mimics structure and function of human soft tissue with endothelial cell-coated larger vessels for perfusion and micro-vessel networks within the hydrogel-matrix. Patient-derived neuroblastoma spheroids are added to the matrix during the printing process. The inventors demonstrated that micro-vessels are attracted by and grow into tumor spheroids and that neuroblastoma cells invade the tumor microenvironment as soon as the spheroids disrupt. These investigations have been obtained as a result of the long term stability of the cell culture over e. g. 28 days. With this 3D bioprinted, micro-vascularized neuroblastoma micro-environment model directly printed into the cultivation device, the inventors described a novel medium-throughput platform suitable for reproducibly and stably studying drug response and metastasis for precision medicine approaches.

The features of the invention disclosed in the above description, the drawings and the claims can be of significance individually, in combination or sub-combination for the implementation of the invention in its different embodiments.

## Claims

1. Cultivation device (100), being configured for cultivating biological cell material (1), comprising
- a substrate body (10) having an upper surface (11), a lower surface (12), at least one receptacle cavity (20) with a bottom wall (21) and a circumferential side wall (22), and at least one fluid channel (30) in communication with the at least one receptacle cavity (20), wherein the at least one receptacle cavity (20) has an opening (23) towards the upper surface (11) of the substrate body (10) and is arranged for accommodating the biological cell material (1),
**characterized in that**
- an inner surface of the side wall (22) is provided with anchoring notches (24) being arranged for holding the biological cell material (1) in the at least one receptacle cavity (20),
- the anchoring notches (24) have a radially extending retaining profile, which is formed such that material in the receptacle cavity (20) is trapped in the anchoring notches (24), wherein the anchoring notches (24) have an angular shape providing a narrowing of the anchoring notches (24) towards the inner surface of the side wall (22), and
- the upper surface (11) of the substrate body (10) has a deepened surface section (13) around the opening (23) of the at least one receptacle cavity (20), wherein the deepened surface section (13) is adapted for accommodating a cavity lid sheet (40) covering the opening (23) of the at least one receptacle cavity (20).

2. Cultivation device according to claim 1, wherein
- the anchoring notches (24) have a step shape (25) providing undercut slit openings (26) of the anchoring notches (24) towards the inner surface of the side wall (22).

3. Cultivation device according to one of the foregoing claims, comprising at least one of the features
- the anchoring notches (24) are evenly distributed along the side wall (22),
- the anchoring notches (24) are non-evenly distributed along the side wall (22), and
- the anchoring notches (24) extend in a direction perpendicular to the upper and lower surfaces (11, 12) of the substrate body (10).

4. Cultivation device according to one of the foregoing claims, wherein the deepened surface section (13) has at least one of the features
- the deepened surface section (13) has a rough finish, and
- the deepened surface section (13) has a profile with a deepened trench (15) extending around the opening (23) of the at least one receptacle cavity (20) and opening towards the anchoring notches (24).

5. Cultivation device according to one of the foregoing claims, wherein
- the circumferential side wall (22) has a circumferential bottom recess being located adjacent to the bottom wall (21) and extending in a radial direction in the substrate body (10), wherein the circumferential bottom recess preferably has a profile, which is formed such that material in the sample receptacle is trapped in the circumferential bottom recess.

6. Cultivation device according to one of the foregoing claims, wherein
- a cross-linked hydrogel is arranged in the at least one receptacle cavity (20), wherein portions of the hydrogel are included in the anchoring notches (24), wherein preferably biological cells and/or cell components of the biological cell material (1) are included in the hydrogel.

7. Cultivation device according to one of the foregoing claims, further comprising
- a membrane layer (41), in particular a web of filaments (42), providing the cavity lid sheet (40) and being coupled with the deepened surface section (13) for covering the opening (23) of the at least one receptacle cavity (20).

8. Cultivation device according to one of the foregoing claims, comprising at least one of the features
- at least one of the at least one receptacle cavity (20) and the at least one fluid channel (30) is coated with at least one of biological cells, collagen, laminin and fibronectin,
- the biological cell material (1) comprises at least one of biological cells and cell components,
- the substrate body (10) is bound via the lower surface (12) to a carrier substrate (14), in particular comprising glass,
- the at least one fluid channel (30) is formed in the upper surface (11) of the substrate body (10),
- the at least one fluid channel (30) is formed in the lower surface of the substrate body (10), and
- the at least one fluid channel (30) is formed within the substrate body (10).

9. Method of manufacturing a cultivation device (100) according to one of the foregoing claims, comprising the step:
- creating the substrate body (10) with the at least one receptacle cavity (20), the at least one fluid channel (30), the anchoring notches (24) and the deepened surface section (13) by subtractive manufacturing from a preformed substrate body workpiece (10A) and/or by additive manufacturing.

10. Method according to claim 9, further comprising at least one of
- bonding the substrate body (10) to a carrier substrate,
- subjecting the at least one receptacle cavity (20) and the at least one fluid channel (30), including the anchoring notches (24) and the deepened surface section (13), to a plasma treatment, and
- creating the substrate body (10) by the subtractive manufacturing, including a material removal from a preformed substrate body (10) workpiece, wherein the material removal comprises laser beam-based engraving, in particular being executed with a pulsed laser having a repetition frequency below 400 Hz.

11. Method according to one of the claims 9 to 10, further comprising
- depositing a liquid hydrogel (2), in particular including biological cells and/or cell components of the biological cell material (1), in the at least one receptacle cavity (20), so that the anchoring notches (24) are filled with the liquid hydrogel (2), and
- cross-linking the hydrogel (2) by at least one of irradiation, temperature setting and chemical cross-linking.

12. Method according to claim 11, comprising at least one of the features
- the biological cell material (1) comprises at least one of fibroblasts, endothelial cells, mesothelial cells, smooth muscle cells, organ cells, like liver cells, induced pluripotent stem cells (iPSC) and iPSC-derived cells, mesenchymal stem cells and spheroid cell bodies, or at least one component thereof, and
- the hydrogel (2) comprises at least one natural hydrogel component, like gelatin-methacryloyl (GelMA), alginate, functionalized alginate-YISGR, chitosan, hyaluronic acid, hyaluronic acid metacrylate, collagen, nano-cellulose, silk, synthetic hydrogels, like PEG, PEGDA or PVA, synthetic extracellular proteins, and/or a glycoprotein, like fibrinogen and/or fibronectin.

13. Method according to one of the claims 11 or 12, further comprising preparing at least one hollow vessel structure in the hydrogel (2), deposited in the at least one receptacle cavity (20), by
- depositing a vessel-shaped sacrificial material (3) in the hydrogel (2), wherein the sacrificial material (3) is coupled with the at least one fluid channel (30) and removing the sacrificial material (3) after cross-linking the hydrogel, so that the at least one hollow vessel structure (4) is formed, or
- an additive printing technique that allows fabrication of hollow structures.

14. Method according to one of the claims 9 to 13, further comprising
- coupling a membrane layer (41) providing a cavity lid sheet (40) with the deepened surface section (13) for covering the opening (23) of the at least one receptacle cavity (20), wherein preferably further biological cell material (1) is deposited on the membrane layer (41).

15. Method according to claim 14, comprising at least one of the features
- the membrane layer (41) is a spun web of filaments (42), and
- the membrane layer (41) is coupled with the deepened surface section (13) by an effect of a chemical solvent and/or by a melting process.

16. Method of using a cultivation device according to one of the claims 1 to 8 or a method according to one of the claims 9 to 15, for at least one of
- cultivating biological cells,
- organ-on-chip processes,
- self organization of composites of biological cells,
- ripening of biological cells, investigating degeneration processes of biological cells,
- testing pharmaceutically active substances (e. g. drug testing),
- creating multi-tissue/organ models,
- investigating wound healing processes,
- creating biosensors,
- biohazard detection,
- parasitology investigations,
- creating barrier models (e. g. blood-brain barrier, skin, intestine, lung),
- in vitro vaccination processes by implementation of antigen-presenting cells,
- antigen and vaccine screening,
- mechanobiological investigations,
- investigations of living technical circulation systems,
- creating tumor environments,
- investigations of tumor vascularization, metastasis and adhesion of tumor metastases,
- investigations of aging processes,
- investigations of tissue-specific metabolism, and
- methods of disease modelling.

## Patentansprüche

1. Kultivierungsvorrichtung (100), die zum Kultivieren von biologischem Zellmaterial (1) konfiguriert ist, umfassend
- einen Substratkörper (10), der eine obere Oberfläche (11), eine untere Oberfläche (12), mindestens eine Aufnahmevertiefung (20) mit einer Bodenwand (21) und einer umlaufenden Seitenwand (22), und mindestens einen Fluidkanal (30) aufweist, der mit der mindestens einen Aufnahmevertiefung (20) in Verbindung steht, wobei die mindestens eine Aufnahmevertiefung (20) eine Öffnung (23) zur oberen Oberfläche (11) des Substratkörpers (10) hin aufweist und zum Aufnehmen des biologischen Zellmaterials (1) angeordnet ist,
**dadurch gekennzeichnet, dass**
- eine Innenfläche der Seitenwand (22) mit Verankerungskerben (24) versehen ist, die zum Halten des biologischen Zellmaterials (1) in der mindestens einen Aufnahmevertiefung (20) angeordnet sind,
- die Verankerungskerben (24) ein sich radial erstreckendes Halteprofil aufweisen, das derart ausgebildet ist, dass Material in der Aufnahmevertiefung (20) in den Verankerungskerben (24) eingefangen wird, wobei die Verankerungskerben (24) eine winkelige Form aufweisen, die eine Verengung der Verankerungskerben (24) zur Innenfläche der Seitenwand (22) hin bereitstellt, und
- die obere Oberfläche (11) des Substratkörpers (10) einen vertieften Oberflächenabschnitt (13) um die Öffnung (23) der mindestens einen Aufnahmevertiefung (20) herum aufweist, wobei der vertiefte Oberflächenabschnitt (13) zum Aufnehmen eines Vertiefungsdeckelblatts (40), das die Öffnung (23) der mindestens einen Aufnahmevertiefung (20) abdeckt, angepasst ist.

2. Kultivierungsvorrichtung nach Anspruch 1, wobei
- die Verankerungskerben (24) eine Stufenform (25) aufweisen, die hinterschnittene Schlitzöffnungen (26) der Verankerungskerben (24) zur Innenfläche der Seitenwand (22) hin bereitstellt.

3. Kultivierungsvorrichtung nach einem der vorhergehenden Ansprüche, umfassend mindestens eines der Merkmale
- die Verankerungskerben (24) sind gleichmäßig entlang der Seitenwand (22) verteilt,
- die Verankerungskerben (24) sind ungleichmäßig entlang der Seitenwand (22) verteilt, und
- die Verankerungskerben (24) erstrecken sich in einer Richtung senkrecht zu der oberen und der unteren Oberfläche (11, 12) des Substratkörpers (10).

4. Kultivierungsvorrichtung nach einem der vorhergehenden Ansprüche, wobei der vertiefte Oberflächenabschnitt (13) mindestens eines der Merkmale aufweist
- der vertiefte Oberflächenabschnitt (13) weist eine raue Oberflächenbeschaffenheit auf, und
- der vertiefte Oberflächenabschnitt (13) weist ein Profil mit einer vertieften Rinne (15) auf, die sich um die Öffnung (23) der mindestens einen Aufnahmevertiefung (20) herum erstreckt und sich zu den Verankerungskerben (24) hin öffnet.

5. Kultivierungsvorrichtung nach einem der vorhergehenden Ansprüche, wobei
- die umlaufende Seitenwand (22) eine umlaufende Bodenausnehmung aufweist, die benachbart zu der Bodenwand (21) angeordnet ist und sich in einer radialen Richtung in dem Substratkörper (10) erstreckt, wobei die umlaufende Bodenausnehmung vorzugsweise ein Profil aufweist, das derart ausgebildet ist, dass Material in der Probenaufnahme in der umlaufenden Bodenausnehmung eingefangen wird.

6. Kultivierungsvorrichtung nach einem der vorhergehenden Ansprüche, wobei
- ein vernetztes Hydrogel in der mindestens eine Aufnahmevertiefung (20) angeordnet ist, wobei Abschnitte des Hydrogels in den Verankerungskerben (24) enthalten sind, wobei vorzugsweise biologische Zellen und/oder Zellbestandteile des biologischen Zellmaterials (1) in dem Hydrogel enthalten sind.

7. Kultivierungsvorrichtung nach einem der vorhergehenden Ansprüche, ferner umfassend
- eine Membranschicht (41), insbesondere eine Bahn aus Filamenten (42), die das Vertiefungsdeckelblatt (40) bereitstellt und mit dem vertieften Oberflächenabschnitt (13) gekoppelt ist, um die Öffnung (23) der mindestens einen Aufnahmevertiefung (20) abzudecken.

8. Kultivierungsvorrichtung nach einem der vorhergehenden Ansprüche, umfassend mindestens eines der Merkmale
- mindestens eines von der mindestens eine Aufnahmevertiefung (20) und dem mindestens einen Fluidkanal (30) ist mit mindestens einem von biologischen Zellen, Kollagen, Laminin und Fibronektin beschichtet,
- das biologische Zellmaterial (1) umfasst mindestens eines von biologischen Zellen und Zellbestandteilen,
- der Substratkörper (10) ist über die untere Oberfläche (12) an ein Trägersubstrat (14), insbesondere umfassend Glas, gebunden,
- der mindestens eine Fluidkanal (30) ist in der oberen Oberfläche (11) des Substratkörpers (10) ausgebildet,
- der mindestens eine Fluidkanal (30) ist in der unteren Oberfläche des Substratkörpers (10) ausgebildet, und
- der mindestens eine Fluidkanal (30) ist in dem Substratkörper (10) ausgebildet.

9. Verfahren zur Herstellung einer Kultivierungsvorrichtung (100) nach einem der vorhergehenden Ansprüche, umfassend den Schritt:
- Erzeugen des Substratkörpers (10) mit der mindestens eine Aufnahmevertiefung (20), dem mindestens einen Fluidkanal (30), den Verankerungskerben (24) und dem vertieften Oberflächenabschnitt (13) durch subtraktive Fertigung aus einem vorgeformten Substratkörper-Werkstück (10A) und/oder durch additive Fertigung.

10. Verfahren nach Anspruch 9, ferner umfassend mindestens eines von
- Bonden des Substratkörpers (10) an ein Trägersubstrat,
- Unterziehen der mindestens einen Aufnahmevertiefung (20) und des mindestens einen Fluidkanals (30), einschließlich der Verankerungskerben (24) und des vertieften Oberflächenabschnitts (13), einer Plasmabehandlung, und
- Erzeugen des Substratkörpers (10) durch die subtraktive Fertigung, einschließlich eines Materialabtrags von einem vorgeformten Substratkörper-(10)-Werkstück, wobei der Materialabtrag ein laserstrahlbasiertes Gravieren umfasst, das insbesondere mit einem gepulsten Laser mit einer Wiederholfrequenz unter 400 Hz ausgeführt wird.

11. Verfahren nach einem der Ansprüche 9 bis 10, ferner umfassend
- Einbringen eines flüssigen Hydrogels (2), insbesondere biologische Zellen und/oder Zellbestandteile des biologischen Zellmaterials (1) enthaltend, in die mindestens eine Aufnahmevertiefung (20), so dass die Verankerungskerben (24) mit dem flüssigen Hydrogel (2) gefüllt werden, und
- Vernetzen des Hydrogels (2) durch mindestens eines von Bestrahlung, Temperatureinstellung und chemischer Vernetzung.

12. Verfahren nach Anspruch 11, umfassend mindestens eines der Merkmale
- das biologische Zellmaterial (1) umfasst mindestens eines von Fibroblasten, Endothelzellen, Mesothelzellen, glatten Muskelzellen, Organzellen, wie Leberzellen, induzierten pluripotenten Stammzellen (iPSC) und von iPSC abgeleiteten Zellen, mesenchymalen Stammzellen und sphäroiden Zellkörpern, oder mindestens eine Komponente davon, und
- das Hydrogel (2) umfasst mindestens einen natürlichen Hydrogelbestandteil, wie Gelatine-Methacryloyl (GelMA), Alginat, funktionalisiertes Alginat-YISGR, Chitosan, Hyaluronsäure, Hyaluronsäuremethacrylat, Kollagen, Nanozellulose, Seide, synthetische Hydrogele, wie PEG, PEGDA oder PVA, synthetische extrazelluläre Proteine und/oder ein Glykoprotein, wie Fibrinogen und/oder Fibronektin.

13. Verfahren nach einem der Ansprüche 11 oder 12, ferner umfassend das Vorbereiten mindestens einer Hohlgefäßstruktur in dem Hydrogel (2), das in die mindestens einen Aufnahmevertiefung (20) eingebracht ist, durch
- Einbringen eines gefäßförmigen Opfermaterials (3) in das Hydrogel (2), wobei das Opfermaterial (3) mit dem mindestens einen Fluidkanal (30) gekoppelt ist, und Entfernen des Opfermaterials (3) nach dem Vernetzen des Hydrogels, sodass die mindestens eine Hohlgefäßstruktur (4) gebildet wird, oder
- eine additive Drucktechnik, die die Herstellung von Hohlstrukturen ermöglicht.

14. Verfahren nach einem der Ansprüche 9 bis 13, ferner umfassend
- Koppeln einer Membranschicht (41), die ein Vertiefungsdeckelblatt (40) bereitstellt, mit dem vertieften Oberflächenabschnitt (13), um die Öffnung (23) der mindestens einen Aufnahmevertiefung (20) abzudecken, wobei vorzugsweise weiteres biologisches Zellmaterial (1) auf der Membranschicht (41) aufgebracht wird.

15. Verfahren nach Anspruch 14, umfassend mindestens eines der Merkmale
- die Membranschicht (41) ist eine gesponnene Bahn aus Filamenten (42), und
- die Membranschicht (41) ist mit dem vertieften Oberflächenabschnitt (13) durch die Wirkung eines chemischen Lösungsmittels und/oder durch einen Schmelzprozess gekoppelt.

16. Verfahren zur Verwendung einer Kultivierungsvorrichtung nach einem der Ansprüche 1 bis 8 oder eines Verfahrens nach einem der Ansprüche 9 bis 15, für mindestens eines von
- Kultivieren von biologischen Zellen,
- Organ-on-Chip-Prozessen,
- Selbstorganisation von Kompositen biologischer Zellen,
- Reifung von biologischen Zellen, Untersuchung von Degenerationsprozessen biologischer Zellen,
- Testen pharmazeutisch wirksamer Substanzen (z. B. Arzneimitteltests),
- Erstellen von Multi-Gewebe-/Organmodellen,
- Untersuchen von Wundheilungsprozessen,
- Erstellen von Biosensoren,
- Biogefahrendetektion,
- parasitologischen Untersuchungen,
- Erstellen von Barrieremodellen (z. B. Blut-Hirn-Schranke, Haut, Darm, Lunge),
- In-vitro-Impfungsprozessen durch Implementierung von antigenpräsentierenden Zellen,
- Antigen- und Impfstoff-Screening,
- mechanobiologischen Untersuchungen,
- Untersuchungen von lebenden technischen Kreislaufsystemen,
- Erstellen von Tumorumgebungen,
- Untersuchungen der Tumorvaskularisierung, Metastasierung und Adhäsion von Tumormetastasen,
- Untersuchungen von Alterungsprozessen,
- Untersuchungen von gewebespezifischem Stoffwechsel, und
- Verfahren zur Krankheitsmodellierung.

## Revendications

1. Dispositif de culture (100), qui est conçu pour cultiver une matière cellulaire biologique (1), comprenant
- un corps de substrat (10) présentant une surface supérieure (11), une surface inférieure (12), au moins une cavité de réceptacle (20) comportant une paroi inférieure (21) et une paroi latérale périphérique (22), et au moins un canal de fluide (30) en communication avec l'au moins une cavité de réceptacle (20), la cavité de réceptacle (20) présentant une ouverture (23) vers la surface supérieure (11) du corps de substrat (10) et étant agencée pour recevoir la matière cellulaire biologique (1),
**caractérisé en ce que**
- une surface intérieure de la paroi latérale (22) est pourvue d'encoches d'ancrage (24) destinées à maintenir la matière cellulaire biologique (1) dans l'au moins une cavité de réceptacle (20),
- les encoches d'ancrage (24) ont un profil de retenue s'étendant radialement, qui est formé de telle sorte que le matériau dans la cavité de réceptacle (20) soit piégé dans les encoches d'ancrage (24), les encoches d'ancrage (24) ayant une forme angulaire fournissant un rétrécissement des encoches d'ancrage (24) vers la surface intérieure de la paroi latérale (22), et
- la surface supérieure (11) du corps de substrat (10) présente une section de surface approfondie (13) autour de l'ouverture (23) de l'au moins une cavité de réceptacle (20), la section de surface approfondie (13) étant conçue pour recevoir une feuille de couvercle de cavité (40) recouvrant l'ouverture (23) de l'au moins une cavité de réceptacle (20).

2. Dispositif de culture selon la revendication 1, dans lequel
- les encoches d'ancrage (24) ont une forme étagée (25) qui forme des ouvertures fendues en contre-dépouille (26) des encoches d'ancrage (24) vers la surface intérieure de la paroi latérale (22).

3. Dispositif de culture selon l'une des revendications précédentes, comprenant au moins l'une des caractéristiques
- les encoches d'ancrage (24) sont uniformément réparties le long de la paroi latérale (22),
- les encoches d'ancrage (24) ne sont pas réparties uniformément le long de la paroi latérale (22), et
- les encoches d'ancrage (24) s'étendent dans une direction perpendiculaire aux surfaces supérieure et inférieure (11, 12) du corps de substrat (10).

4. Dispositif de culture selon l'une des revendications précédentes, dans lequel la section de surface approfondie (13) présente au moins l'une des caractéristiques
- la section de surface approfondie (13) a une finition rugueuse, et
- la section de surface approfondie (13) présente un profil comportant une tranchée approfondie (15) s'étendant autour de l'ouverture (23) de l'au moins une cavité de réceptacle (20) et s'ouvrant vers les encoches d'ancrage (24).

5. Dispositif de culture selon l'une des revendications précédentes, dans lequel
- la paroi latérale circonférentielle (22) présente un évidement inférieur circonférentiel qui est situé adjacent à la paroi inférieure (21) et s'étendant dans une direction radiale dans le corps de substrat (10), l'évidement inférieur circonférentiel présentant de préférence un profil, qui est formé de sorte que de la matière contenue dans le réceptacle d'échantillon soit piégée dans l'évidement inférieur circonférentiel.

6. Dispositif de culture selon l'une des revendications précédentes, dans lequel
- un hydrogel réticulé est disposé dans l'au moins une cavité de réceptacle (20), des parties de l'hydrogel étant incluses dans les encoches d'ancrage (24), de préférence des cellules biologiques et/ou des composants cellulaires de la matière cellulaire biologique (1) étant inclus dans l'hydrogel.

7. Dispositif de culture selon l'une des revendications précédentes, comprenant en outre
- une couche de membrane (41), en particulier une nappe de filaments (42), formant la feuille de couvercle de cavité (40) et qui est couplée à la section de surface approfondie (13) pour recouvrir l'ouverture (23) de l'au moins une cavité de réceptacle (20).

8. Dispositif de culture selon l'une des revendications précédentes, comprenant au moins l'une des caractéristiques
- au moins un(e) de l'au moins une cavité de réceptacle (20) et de l'au moins un canal de fluide (30) est revêtu(e) par au moins l'un parmi des cellules biologiques, du collagène, de la laminine et de la fibronectine,
- la matière cellulaire biologique (1) comprend au moins l'un parmi des cellules biologiques et des composants cellulaires,
- le corps de substrat (10) est lié par la surface inférieure (12) à un substrat de support (14), en particulier comprenant du verre,
- l'au moins un canal de fluide (30) est formé dans la surface supérieure (11) du corps de substrat (10),
- l'au moins un canal de fluide (30) est formé dans la surface inférieure du corps de substrat (10), et
- l'au moins un canal de fluide (30) est formé à l'intérieur du corps de substrat (10).

9. Procédé de fabrication d'un dispositif de culture (100) selon l'une des revendications précédentes, comprenant l'étape :
- création du corps de substrat (10) comportant l'au moins une cavité de réceptacle (20), l'au moins un canal de fluide (30), les encoches d'ancrage (24) et la section de surface approfondie (13) par fabrication soustractive à partir d'une pièce de corps de substrat préformée (10A) et/ou par fabrication additive.

10. Procédé selon la revendication 9, comprenant en outre au moins l'un parmi
- la liaison du corps de substrat (10) à un substrat de support,
- la soumission de l'au moins une cavité de réceptacle (20) et l'au moins un canal de fluide (30), y compris les encoches d'ancrage (24) et la section de surface approfondie (13), à un traitement au plasma, et
- la réalisation du corps de substrat (10) par la fabrication soustractive, y compris un enlèvement de matière à partir d'une pièce de corps de substrat (10) préformée, l'enlèvement de matière comprenant une gravure à base de faisceau laser, en particulier qui est exécutée avec un laser pulsé ayant une fréquence de répétition inférieure à 400 Hz.

11. Procédé selon l'une des revendications 9 à 10, comprenant en outre
- le dépôt d'un hydrogel liquide (2), en particulier comprenant des cellules biologiques et/ou des composants cellulaires de la matière cellulaire biologique (1), dans l'au moins une cavité de réceptacle (20), de sorte que les encoches d'ancrage (24) soient remplies avec l'hydrogel liquide (2), et
- la réticulation de l'hydrogel (2) par au moins l'un parmi une irradiation, un ajustement de la température et une réticulation chimique.

12. Procédé selon la revendication 11, comprenant au moins l'une des caractéristiques
- la matière cellulaire biologique (1) comprend au moins l'un parmi des fibroblastes, des cellules endothéliales, des cellules mésothéliales, des cellules musculaires lisses, des cellules d'organes, comme des cellules hépatiques, des cellules souches pluripotentes induites (iPSC) et des cellules dérivées d'iPSC, des cellules souches mésenchymateuses et des corps cellulaires sphéroïdes, ou au moins un composant correspondant, et
- l'hydrogel (2) comprend au moins un composant hydrogel naturel, comme la gélatine-méthacryloyle (GelMA), l'alginate, l'alginate fonctionnalisé-YISGR, le chitosane, l'acide hyaluronique, le méthacrylate d'acide hyaluronique, le collagène, la nano-cellulose, la soie, les hydrogels synthétiques, comme le PEG, le PEGDA ou le PVA, les protéines extracellulaires synthétiques et/ou une glycoprotéine, comme le fibrinogène et/ou la fibronectine.

13. Procédé selon l'une des revendications 11 ou 12, comprenant en outre la préparation d'au moins une structure de récipient creuse dans l'hydrogel (2), déposée dans l'au moins une cavité de réceptacle (20), par
- dépôt d'un matériau sacrificiel (3) en forme de récipient dans l'hydrogel (2), le matériau sacrificiel (3) étant couplé avec l'au moins un canal de fluide (30) et élimination du matériau sacrificiel (3) après réticulation de l'hydrogel, de manière à former au moins une structure de récipient creuse (4), ou
- une technique d'impression additive qui permet la fabrication de structures creuses.

14. Procédé selon l'une des revendications 9 à 13, comprenant en outre
- couplage d'une couche de membrane (41) fournissant une feuille de couvercle de cavité (40) avec la section de surface approfondie (13) pour recouvrir l'ouverture (23) de l'au moins une cavité de réceptacle (20), une autre matière cellulaire biologique (1) étant de préférence déposée sur la couche de membrane (41).

15. Procédé selon la revendication 14, comprenant au moins l'une des caractéristiques
- la couche de membrane (41) est une nappe filée de filaments (42), et
- la couche de membrane (41) est couplée à la section superficielle approfondie (13) par un effet d'un solvant chimique et/ou par un processus de fusion.

16. Procédé d'utilisation d'un dispositif de culture selon l'une des revendications 1 à 8 ou d'un procédé selon l'une des revendications 9 à 15, pour au moins l'un parmi
- culture de cellules biologiques,
- processus d'organe sur puce,
- auto-organisation de composites de cellules biologiques,
- maturation de cellules biologiques, étude de processus de dégénérescence des cellules biologiques,
- test de substances pharmaceutiquement actives (par exemple, test de médicaments),
- création de modèles multi-tissus/d'organes,
- étude de processus de cicatrisation des plaies,
- création de biocapteurs,
- détection des risques biologiques,
- investigations parasitologiques,
- création de modèles de barrière (par exemple, barrière hémato-encéphalique, peau, intestin, poumon),
- processus de vaccination *in vitro* par mise en œuvre de cellules présentatrices d'antigènes,
- dépistage d'antigènes et de vaccins,
- études mécanobiologiques,
- études de systèmes de circulation technique vivante,
- création d'environnements tumoraux,
- études de la vascularisation tumorale, des métastases et de l'adhérence des métastases tumorales,
- études des processus de vieillissement,
- études du métabolisme spécifique à un tissu, et
- procédés de modélisation de maladies.
